# FASCICULE DE BREVET EUROPEEN

(11) **EP 0 916 327 B1**
(45) Date de publication et mention de la délivrance du brevet: **17.09.2003**
(21) Numéro de dépôt: 98490029.0
(22) Date de dépôt: 04.11.1998
(51) Int. Cl.: A61F 13/15

(54) **Article d'hygiène a panneaux respirables en non-tisse**
Aborbierende Artikel mit luftdurchlässigen Vliesstoffseitenwänden
Absorbent article having breathable nonwoven side panels

(30) Priorité: 04.11.1997 FR 9714116
(43) Date de publication de la demande: 19.05.1999
(73) Titulaire: Proteco, 59494 Petite Forêt (FR)
(72) Inventeur: Brutin, Jean-Marc, 59780 Camphin en Pevele (FR)
(74) Mandataire: Tanty, François

(56) Documents cités:
- EP-A- 0 403 832
- EP-A- 0 409 149
- WO-A-97/30671
- US-A- 5 571 096

## Description

La présente invention concerne un article d'hygiène, notamment couche-culotte, qui est pourvu de panneaux latéraux respirables, c'est-à-dire aptes à permettre le passage de l'air, tout en empêchant éventuellement le passage de liquide entre l'intérieur et l'extérieur de l'article.

Une couche-culotte comprend de manière classique une feuille extérieure (backsheet) imperméable aux liquides , une feuille intérieure (coverstock) perméable aux liquides et un matelas absorbant disposé entre les deux dites feuilles. Un tel article d'hygiène a pour fonction de contenir l'urine et les matières fécales émises par l'utilisateur tout en isolant ces matières de l'environnement extérieur. C'est pourquoi la feuille extérieure est imperméable aux liquides ; il s'agit habituellement d'une feuille en polyéthylène qui est également imperméable à l'air et à la vapeur d'eau ce qui rend la couche-culotte inconfortable au porter.

On a déjà proposé différentes possibilités pour rendre une telle couche-culotte plus confortable en mettant en oeuvre comme feuille extérieure une feuille qui soit respirable, c'est-à-dire qui tout en étant imperméable aux liquides puisse permettre les échanges d'air et de vapeur d'eau. On a par exemple proposé l'utilisation d'un film microporeux ou encore d'un film dans un matériau imperméable mais présentant des perforations ayant une figuration telle qu'elles permettent le passage des vapeurs tout en empêchant le passage des liquides.

Dans le document WO.97/10788, on a proposé un article d'hygiène absorbant ayant des panneaux de côté respirables. Dans ce document est décrit un article d'hygiène du type couche-culotte, dont la feuille extérieure est réalisée dans un matériau imperméable, notamment en polyéthylène , et qui comprend une zone centrale correspondant sensiblement à la zone dans laquelle ladite feuille est en contact avec le matelas absorbant et deux zones latérales, correspondant aux panneaux de côté. Dans ces zones latérales , la feuille extérieure est perforée de manière à la rendre perméable à l'air. Dans une version préférée , une feuille perméable aux liquides et à l'air est associée à la feuille imperméable aux liquides perforée sur la face intérieure de celle-ci pour former un complexe respirable dans les zones perforées de la feuille imperméable.

Selon le demandeur , la solution préconisée par le document WO.97/10788 n'est pas totalement satisfaisante. Selon la version de base la feuille imperméable aux liquides qui est dans un matériau plastique perforé est en contact direct avec la peau de l'utilisateur, ce qui n'est guère confortable au porter. Pour remédier à cet inconvénient, le document précité propose de réaliser un complexe entre cette feuille plastique imperméable perforée avec une feuille complémentaire perméable aux liquides et à l'air. Cette solution est plus complexe et plus coûteuse à mettre en oeuvre.

Le document WO.97/30671 concerne une culotte jetable avec des panneaux de côté qui ont la particularité d'être élastiques de manière à permettre la bonne application de la culotte sur le corps de l'utilisateur. Ces panneaux de côté sont d'une structure complexe, étant constitués d'une superposition de plusieurs couches dont l'une est élastique.

Le document US.5,246,432 concerne une couche-culotte avec des panneaux de côté non élastiques , mais qui sont réalisés dans un matériau complexe formé de la superposition d'une première feuille absorbant les liquides, et d'une seconde feuille imperméable au liquide et perméable à l'humidité. C'est d'ailleurs le même matériau complexe qui , prolongé, sert à constituer les barrières fécales. Dans la construction de la couche-culotte, la face absorbante du matériau complexe est en contact avec la peau de l'usager.

Le brevet US BUELL 4,636,207 décrit une couche-culotte à usage unique comportant un matelas absorbant et, latéralement de chaque côté de ce matelas absorbant, des bandes perméables à la vapeur au moins sur une partie de leur surface.

La demande de brevet EP 403 832 décrit une couche culotte comportant des panneaux latéraux présentant une structure composite avec une couche supérieure absorbante en contact avec la peau et une couche arrière formant barrière aux liquides et perméable à l'air.

Le brevet US 5 571 096 décrit une couche culotte comportant une feuille amère. constituée par un film plastique fin imperméable aux liquides et une feuille supérieure perméable aux liquides.

Le but que s'est fixé le demandeur est de proposer une couche-culotte à panneaux de côté qui soit d'une structure particulièrement simple et qui soit néanmoins confortable au porter.

Ce but est parfaitement atteint par la couche-culotte de l'invention qui, de manière connue comprend une feuille extérieure imperméable aux liquides, une feuille intérieure perméable aux liquides et un matelas absorbant disposé entre les deux dites feuilles ; les feuilles extérieure et intérieure ont une forme rectangulaire, sont solidarisées l'une à l'autre selon leurs bords longitudinaux pour former avec le matelas absorbant le corps central de la couche-culotte ; de plus elles comportent des panneaux de côté non-élastiques qui sont fixés latéralement au corps central selon des zones de fixation, de part et d'autre des grands côtés du corps central et , au moins, selon les portions avant et arrière de celui-ci.

De manière caractéristique, selon l'invention, chacun des panneaux de côté est exclusivement en non-tissé, perméable à l'air réalisé dans un matériau hydrophobe.

Ainsi, selon la disposition particulière de l'invention, la constitution de chaque panneau latéral est suffisante pour apporter le confort à l'usager, sans être une source de fuite de liquide.

Selon un mode préféré de réalisation d'une couche-culotte comportant des barrières fécales, chaque barrière est en non-tissé, perméable à l'air, réalisée dans un matériau hydrophobe , de préférence formée par un prolongement du panneau de côté au-delà de la zone de fixation au corps central. Contrairement à l'enseignement du document US.5,246,432, dans ce cas , la barrière fécale est constituée d'un seul matériau qui n'est pas à proprement parler imperméable aux liquides; cependant selon le demandeur , le fait que le non-tissé soit dans un matériau hydrophobe permet à celui-ci de jouer suffisamment le rôle de barrière aux liquides.

Avantageusement la couche-culotte de l'invention comprend également un non-tissé extérieur, perméable à l'air et réalisé dans un matériau hydrophobe, qui est fixé extérieurement sur chacun des panneaux de côté , depuis les zones de fixation au corps central desdits panneaux. Cette disposition particulière vise simplement à améliorer la résistance mécanique des panneaux de côté , qui gardent la même constitution mais qui sont dans ce cas doublés.

Dans un mode particulièrement avantageux de réalisation, le non-tissé extérieur s'étend et est fixé à la fois sur les deux panneaux de côté et sur la feuille extérieure imperméable. La présence de ce non-tissé extérieur sur la feuille extérieure imperméable donne à la couche-culotte une apparence et un toucher moins plastique, plus textile et donc plus agréable. Mais de plus la présence des fibres du non-tissé extérieur permet, lorsque la fermeture de la couche-culotte est réalisée ave des pattes d'attache latérales pourvues de crochets, de refermer facilement sur elle-même la couche-culotte après usage , les crochets venant se prendre dans les fibres du non-tissé extérieur.

Le non-tissé extérieur est formé par une bande qui , après découpe , a la même configuration extérieure à plat que celle de la couche-culotte. Elle peut également être formée à partir d'une bande qui après découpe et pliage , forme à la fois le non-tissé extérieur et les deux panneaux de côté, voire même les deux barrières fécales.

La présente invention sera mieux comprise à la lecture de la description qui va être faite de plusieurs exemples de réalisation de couches-culottes à panneaux de côté respirables en non-tissé perméable à l'air et réalisé dans un matériau hydrophobe illustrés par le dessin annexé dans lequel :
- La figure 1 est une représentation schématique partielle en plan , en vue de dessous d'un premier exemple de couche-culotte,
- La figure 2 est une représentation schématique en coupe selon l'axe II-II de la couche-culotte de la figure 1,
- La figure 3 est une représentation schématique en coupe d'un deuxième exemple de couche-culotte,

Une couche-culotte, comporte de manière bien connue un matelas absorbant , réalisé notamment à base de fibres du type fluff avec ou sans matériau super-absorbant, ce matelas étant pris en sandwich entre d'une part une feuille plastique imperméable aux liquides et généralement à l'air et d'autre part une feuille perméable aux liquides et à l'air. La seconde feuille perméable est en contact direct avec la peau de l'usager, elle est dénommée feuille intérieure. La première feuille, à l'opposé du corps de l'usager est dénommée feuille extérieure.

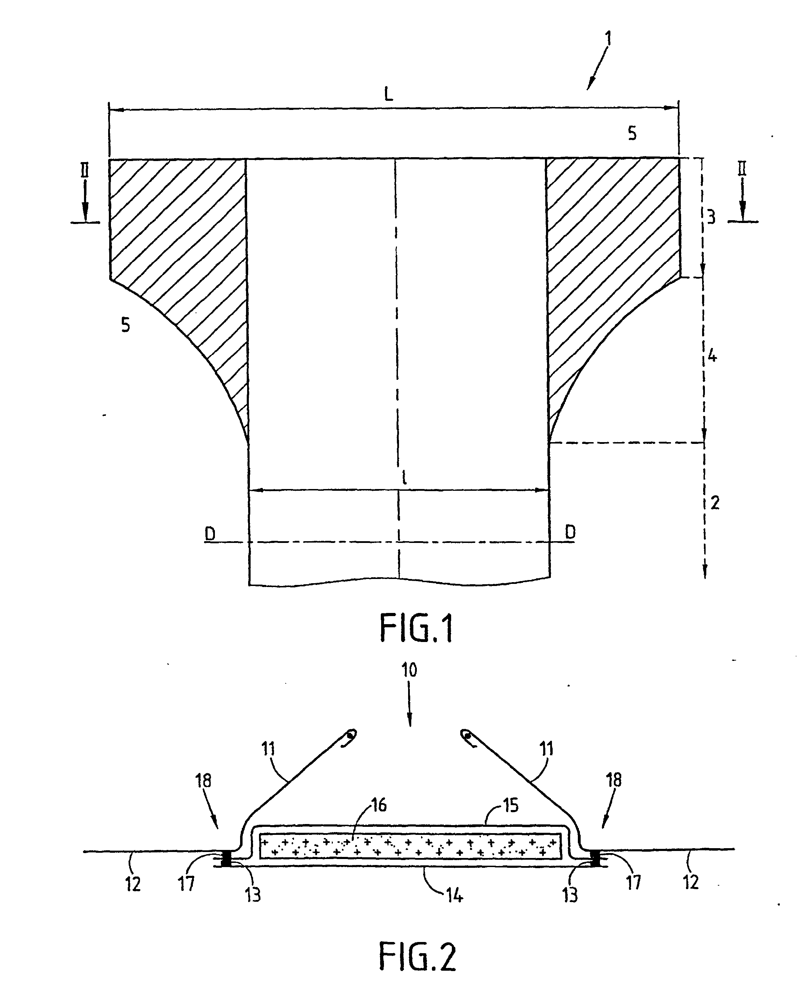

Sur la figure 1 on a représenté la configuration générale d'une couche-culotte 1 globalement en forme de sablier avec une zone 2 rétrécie correspondant à l'entrejambe, une zone 3 élargie à proximité du bord transversal de la couche 1 correspondant à la portion avant (et arrière) de la couche 1 et une zone 4 intermédiaire dans laquelle la couche 1 passe de la largeur l de la zone rétrécie 2 à la largeur L de la portion avant 3.

Lors de la mise en place de la couche sur l'usager, la première zone 2 se trouve placée dans l'entrejambe, tandis que la deuxième zone 3 se trouve au niveau de la taille. Les parties hachurées sur la figure 1 qui correspondent, pour les zones 3 et 4 à ce qui va au-delà de la largeur l de l'entrejambe constituent les panneaux de côté 5.Ces panneaux de côté 5 sont destinés à recouvrir les flancs de l'usager au-dessus des cuisses et permettent la fermeture sur elle-même de la couche-culotte par recouvrement du panneau arrière par le panneau avant et mise en oeuvre de moyens de fermeture adaptés , qu'ils soient du type adhésif ou du type mécanique à crochets et à boucles.

La feuille extérieure imperméable est rectangulaire de largeur l, recouvrant le matelas absorbant, également rectangulaire, ayant une largeur légèrement inférieure à l. Les panneaux de côté, respirables, sont quant à eux constitués exclusivement d'un non-tissé non-élastiqué, perméable à l'air, et réalisés dans un matériau hydrophobe.

Dans le premier exemple de réalisation qui est illustré à la figure 2 d'une couche-culotte 10, la feuille intérieure 15 perméable à l'air, s'étend au-dessus du matelas absorbant 16 ; la feuille extérieure 14 et la feuille intérieure 15 sont fixées l'une à l'autre selon deux lignes longitudinales de colle 13 formant les zones longitudinales de fixation, à proximité immédiate des bords 16a du matelas absorbant 16.

De plus, la couche-culotte 10 comporte des barrières fécales 11. Ce type d'élément est bien connu dans le domaine des couches-culottes; une barrière fécale est une pièce qui est généralement rectangulaire et qui est fixée, selon l'un de ses bords longitudinaux et selon ses deux bords transversaux le long du matelas absorbant qui comporte selon le second bord longitudinal un moyen élastique ; lors de la fabrication en continu de la couche , l'élastique est à l'état tendu et la pièce est à plat au-dessus de la feuille intérieure ; lorsque la couche est sur l'usager, l'élastique se détend et le côté longitudinal muni de cet élastique se relève par rapport au matelas absorbant, créant pour les matières fécales une barrière latérale.

Les barrières fécales 11 sont elles-mêmes réalisées dans un non-tissé perméable à l'air mais dans un matériau hydrophobe ; les panneaux de côté 12 respirables sont constitués par des prolongements desdites barrières fécales 11 dans les zones 3 et 4 de la couche-culotte 10.

Un second jeu de deux lignes de colle 17, venant par exemple se superposer aux premières lignes de colle 13, permet de fixer sur la feuille intérieure 15 les bandes de non-tissé 18 qui, de part et d'autre de ces lignes de colle 17, constituent d'une part les barrières fécales 11 et d'autre part les panneaux de côté respirables 12.

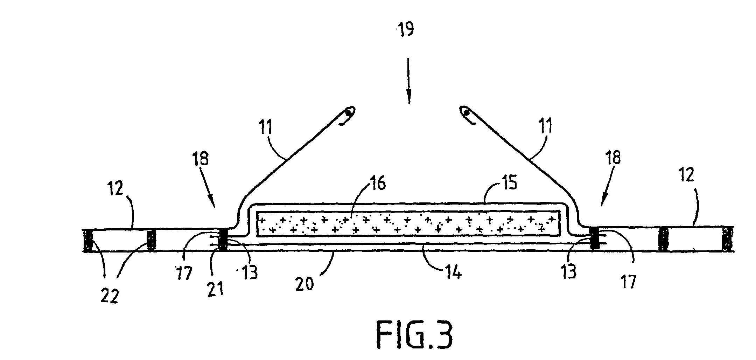

Dans le deuxième exemple de réalisation d'une couche-culotte, qui est illustrée à la figure 3, on a repris les mêmes références pour les éléments communs au premier exemple. En effet ce second mode de réalisation ne se distingue du premier qu'en ce qu'un non-tissé extérieur 20 s'étend sur toute la surface de la couche-culotte 19, à la fois sur la feuille extérieure 14 et sur les deux panneaux de côté 12. Ce non-tissé extérieur 20 est, comme les panneaux de côté 12, un non-tissé perméable à l'air et réalisé dans un matériau hydrophobe. Il est fixé à la feuille extérieure 14 et aux panneaux de côté 12 par collage, en particulier selon un troisième jeu 21 de lignes de colle longitudinales, venant sensiblement se superposer aux deux lignes précédentes 13,17 dans les zones de fixation longitudinales du corps central. Le collage du non-tissé extérieur 20 et des panneaux de côté 12 se fait également par des lignes longitudinales de colle 22, voire même l'application de points de colle régulièrement répartis.

Il est à noter que le non-tissé extérieur 20 qui peut être dans le même matériau que les deux panneaux de côté 12 peut également servir à constituer lesdits panneaux 12 et les deux barrières fécales 11 par découpe et pliage sur la machine de production. Dans ce mode de réalisation, une seule feuille de non-tissé permet de réaliser à la fois les deux barrières fécales, les deux panneaux de côté respirables et la couverture de la feuille imperméable extérieure.

La présence de ce non-tissé extérieur 20 permet d'assurer une résistance mécanique suffisante pour les panneaux de côté 12, en utilisant un non-tissé de faible grammage. De plus le fait que le non-tissé extérieur 20 recouvre la feuille imperméable en plastique 14 permet de réaliser facilement la fermeture sur elle-même après usage dans le cas où les moyens de fermeture de la couche lors de la mise en place sur l'usager sont des moyens d'attache mécanique du type à crochets à boucles, les pattes latérales d'attache disposées de part et d'autre des panneaux de côté arrière étant munis de crochets. En effet dans ce cas les crochets peuvent venir s'accrocher dans les fibres constitutives du non-tissé extérieur 20. Même si ce non-tissé extérieur 20 est peu pelucheux et de faible grammage, cet accrochage sera néanmoins suffisant pour permettre cette fermeture. Par ailleurs la présence de ce non-tissé extérieur 20 donne à la couche-culotte 19 une apparence et un toucher plus doux, comparativement au film plastique imperméable 14.

## Revendications

1. Couche-culotte (10, 19) comprenant une feuille extérieure (14) imperméable aux liquides, une feuille intérieure (15) perméable aux liquides et un matelas absorbant (16) disposé entre les deux dites feuilles (14, 15), les feuilles extérieure (14) et intérieure (15) ayant une forme rectangulaire, étant solidarisées l'une à l'autre selon leurs bords longitudinaux pour former avec le matelas absorbant (16) le corps central de la couche-culotte (10, 19), la couche comportant des panneaux de côté (12) avant et arrière non-élastiqués, fixés latéralement au corps central selon des zones de fixation, de part et d'autre des grands côtés du corps central et, au moins, selon les portions avant et arrière de celui-ci, ces panneaux étant destinés à permettre la fermeture sur elle-même de la couche-culotte, couche-culotte **caractérisée en ce que** chacun des panneaux de côté (12) est exclusivement en non-tissé, perméable à l'air, réalisé dans un matériau hydrophobe.

2. Couche-culotte (10) selon la revendication 1 comportant des barrières fécales (11), **caractérisée en ce que** chaque barrière (11) est en non-tissé, perméable à l'air, réalisée dans un matériau hydrophobe.

3. Couche-culotte (10) selon la revendication 2, **caractérisée en ce que** chaque barrière (11) est de préférence formée par un prolongement du panneau de côté (12) au-delà de la zone de fixation (17) au corps central.

4. Couche-culotte (19) selon l'une des revendications 1 à 3, **caractérisée en ce qu'**elle comprend également un non-tissé extérieur (20), perméable à l'air et réalisé dans un matériau hydrophobe, qui est fixé extérieurement sur chacun des panneaux de côté (12), depuis les zones de fixation (13, 17) au corps central desdits panneaux (12).

5. Couche-culotte selon la revendication 4, **caractérisée en ce que** le non-tissé extérieur (20) s'étend et est fixé à la fois sur les deux panneaux de côté (12) et sur la feuille extérieure imperméable (14).

6. Couche-culotte selon la revendication 5, **caractérisée en ce qu'**une bande de non-tissé perméable à l'air et réalisée en un matériau hydrophobe forme, après découpe et pliage, à la fois le non-tissé extérieur (20) et les deux panneaux de côté (12), voire même les deux barrières fécales (11).

7. Couche-culotte (19) comprenant:
- un non-tissé extérieur (20) perméable à l'air et hydrophobe, s'étendant sur toute la surface de la couche-culotte à la fois sur une feuille imperméable (14) et sur des panneaux de côté avant et arrière (12),
- une feuille intérieure (15) perméable aux liquides, et
- un matelas absorbant (16) disposé entre la feuille imperméable (14) et la feuille intérieure (15), les feuilles intérieure (15) et imperméable (14) formant avec le matelas absorbant le corps central de la couche-culotte, les panneaux de côté (12) avant et arrière étant non élastiqués, s'étendant de part et d'autre des grands côtés du corps central et au moins selon les portions avant et arrière de celui-ci, les panneaux de côté (12) étant destinés à permettre la fermeture sur elle-même de la couche-culotte, **caractérisée en ce que** chaque panneau de côté (12) est exclusivement constitué par l'assemblage d'une feuille (11, 12), réalisée dans un non-tissé hydrophobe et perméable à l'air, avec le non-tissé extérieur (20), la couche-culotte comportant en outre :
- des barrières fécales (11) réalisées par des prolongements de ladite feuille (11,12).

8. Couche-culotte selon la revendication précédente, **caractérisée par le fait que** les feuilles intérieure (15) et imperméable (14) présentent une forme rectangulaire et sont solidarisées l'une à l'autre selon leurs bords longitudinaux, le matelas (16) ayant également une forme rectangulaire.

## Patentansprüche

1. Windel (10,19) mit einer äußeren flüssigkeitsundurchlässigen Folie (14), einer inneren flüssigkeitsundurchlässigen Folie (15) und einem zwischen diesen Folien (14,15) angeordneten Saugpolster (16), welche äußere (14) und innere Folien (15) eine rechteckige Form haben, und miteinander entlang ihrer Längskanten auf solche Weise verbunden sind, daß sie mit dem Saugpolster (16) das Mittelstück der Windel (10,19) bilden, das mit nicht-elastischen Seitenlaschen (12) versehen ist, die seitlich am Mittelstück entlang der Befestigungsbereiche, beiderseits der langen Ränder des Mittelstücks und zumindest entlang der Bereiche davor und dahinter befestigt sind, wobei diese Laschen dazu dienen, den Verschluss auf der Windel selbst zu ermöglichen; **dadurch gekennzeichnet, daß** jede der Seitenlaschen (12) ausschließlich aus einem nichtgewebten, luftdurchlässigen, wasserabweisenden Material hergestellt ist.

2. Windel (10) gemäß Anspruch 1, die mit Abdeckungen (11) zum Schutz gegen Exkremente versehen ist, **dadurch gekennzeichnet, daß** jede der Schutzabdeckungen (11) aus einem nichtgewebten, luftdurchlässigen, wasserabweisenden Material hergestellt ist.

3. Windel (10) gemäß Anspruch 2, **dadurch gekennzeichnet, daß** jede der Schutzabdeckungen (11) vorzugsweise durch eine Verlängerung der Seitenlasche (12) über den Befestigungsbereich (17) des Mittelstücks hinweg gebildet wird.

4. Windel (19) gemäß einem der Ansprüche 1 bis 3, **dadurch gekennzeichnet, daß** diese ferner eine nichtgewebte, luftdurchlässige Außenschicht (20) aus einem wasserabweisenden Material umfaßt, die außen auf jeder der Seitenlaschen (12) von den Befestigungsbereichen (13,17) bis zum Mittelstück der Seitenlaschen (12) befestigt ist.

5. Windel gemäß Anspruch 4, **dadurch, gekennzeichnet, daß** die nichtgewebte Außenschicht (20) sich zugleich über die zwei Seitenlaschen (12) und über die äußere flüssigkeitsundurchlässige Folie (14) erstreckt und darauf befestigt ist

6. Windel gemäß Anspruch 5, **dadurch gekennzeichnet, daß** ein nichtgewebtes, luftdurchlässiges Band aus einem wasserabweisenden Material nach dem Zuschneiden und Falten gleichzeitig die nichtgewebte Außenschicht (20) und die zwei Seitenlaschen (12) sowie die zwei Abdeckungen (11) zum Schutz gegen Exkremente bildet.

7. Die Windel (19) besteht aus:
- einer, Dichtgewebten, luftdurchlässigen Außenschicht (20) aus wasserabweisendem Material, die sich über die gesamte Fläche de Windel zugleich über eine flüssigkeitsundurchlässige Folie (14) sowie über zwei Seitenlaschen vorne und hinten (12) erstreckt.
- einer inneren, flüssigkeitsdurchlässigen Folie (15) sowie
- einem zwischen der flüssigkeitsundurchlässigen Folie (14) und der inneren flüssigkeitsdurchlässigen Folie (15) angeordneten Saugpolster (16), wobei die innere Folie (15) und die flüssigkeitsundurchlässige Folie (14) zusammen mit dem Saugpolster das Mittelstück der Windel bilden; die nicht-elastischen vorderen und hinteren Seitenlaschen (12) erstrecken sich beiderseits der langen Ränder des Mittelstücks und zumindest entlang der Bereiche davor und dahinter, wobei die Seitenlaschen (12) dazu dienen, den Verschluss auf der Windel selbst zu ermöglichen, was **dadurch gekennzeichnet ist, dass** jede der Seitenlaschen (12) ausschließlich aus der Verbindung einer Folie (11,12) besteht, die aus einem nichtgewebten, wasserabweisenden und luftdurchlässigen Material mit der nichtgewebten Außenschicht (20) hergestellt ist ; die Windel besteht außerdem aus:
- Abdeckungen (11) zum Schutz gegen Exkremente, die durch eine Verlängerung der genannten Folie (11,12) gebildet werden.

8. Windel gemäß dem vorstehenden Patentanspruch, **dadurch gekennzeichnet, daß** die innere Folie (15) und die flüssigkeitsundurchlässige Folie (14) eine rechteckige Form bilden und miteinander entlang ihrer Längskanten verbunden sind, wobei das Saugpolster (16) ebenfalls eine rechteckige Form bilden und miteinander entlang ihrer Längskanten verbunden sind, wobei das Saugpolster (16) ebenfalls eine rechteckige Form bildet.

## Claims

1. Diaper (10,19) comprising an outer sheet (14) impermeable to liquids, an inner sheet (15) permeable to liquids, and an absorbent core (16) arranged between the two said sheets (14, 15), the outer (14) and inner (15) sheets having a rectangular shape, and being joined to each other along their longitudinal edges to form, with the absorbent core (16), the central body of the diaper (10,19), which includes non-elastic side panels (12) fixed laterally to the central body in fixation zones, on either side of the long sides of the central body and at least in the front and rear portions of the latter, said panels being intended to enable the diaper to be closed on itself, **characterised in that** each of the side panels (12) is exclusively non-woven, permeable to air, and made of a hydrophobic material.

2. Diaper (10) according to Claim 1, comprising fecal barriers (11), **characterised in that** each barrier (11) is non-woven, permeable to air, and made of hydrophobic material.

3. Diaper (10) according to Claim 2, **characterised in that** each barrier (11) is preferably formed by a continuation of the side panel (12) beyond the zone of fixation (17) to the central body.

4. Diaper (19) according to one of Claims 1 to 3, **characterised in that** it also comprises an outer non-woven (20), permeable to air and made of a hydrophobic material, which is fixed externally on each of the side panels (12), starting from the zones of fixation (13,17) of the said panels (12) to the central body.

5. Diaper according to Claim 4, **characterised in that** the outer non-woven (20) extends and is fixed at the same time to the two side panels (12) and to the impermeable outer sheet (14).

6. Diaper according to Claim 5, chatacterised in that a web of non-woven, permeable to air and made of a hydrophobic material, forms, after cutting and folding, both the outer non-woven (20) and the two side panels (12), and even the two fecal barriers (11).

7. Diaper (19) comprising:
- an outer non-woven material (20) permeable to air and hydrophobic, extending over the entire surface of the diaper both over an impermeable sheet (14) and over front and rear side panels (12),
- an inner sheet (15) permeable to liquids, and
- an absorbent core (16) disposed between the impermeable sheet (14) and the inner sheet (15), the inner (15) and impermeable (14) sheets forming with the absorbent core the central body of the diaper,
The front and rear side panels (12) being non-elastic, extending on each part of the large sides of the central body and at least along the front and rear portions of the latter, the side panels (12) being intended to enable the diaper to be closed on itself, **characterised in that** each side panel is formed solely by the connection of a sheet (11, 12), produced from a hydrophobic non-woven material permeable to air, to the outer non-woven material (20), the diaper also comprising:
- fecal barriers (11) produced by extensions of the said sheet (11, 12)

8. Diaper according to the preceding claim, **characterised by** the fact that the inner (15) and impermeable (14) sheets having a rectangular shape and are connected to each other along their longitudinal edges, the core (16) also having a rectangular shape.
